# EUROPEAN PATENT APPLICATION

(11) **EP 4 740 932 A2**
(43) Date of publication of application: **13.05.2026**
(21) Application number: 24836311.1
(22) Date of filing: 03.07.2024
(51) Int. Cl.: A61K 8/34, A61Q 19/00, A61Q 19/08, A61K 31/05, A61K 47/10, A61P 17/00, A61P 17/18, A61P 29/00

(54) **COSMETIC COMPOSITION FOR SKIN IMPROVEMENT AND PHARMACEUTICAL COMPOSITION FOR PREVENTING OR TREATING SKIN DAMAGE, EACH CONTAINING HONOKIOL AND POLYOL**

(30) Priority: 03.07.2023 KR 20230085480; 02.07.2024 KR 20240086952
(71) Applicant: Cosmax, Inc., Hwaseong-si, Gyeonggi-do 18622 (KR)
(72) Inventor: YUN, Jieun, Seongnam-si, Gyeonggi-do (KR); KWON, Youngji, Seongnam-si, Gyeonggi-do 13486 (KR); JANG, Subin, Seongnam-si, Gyeonggi-do 13486 (KR); PARK, Jung Yoen, Seongnam-si, Gyeonggi-do 13486 (KR); KYUNG, Seoyeon, Seoul 07376 (KR); KIM, Sol, Seongnam-si, Gyeonggi-do 13486 (KR); PARK, Eunjin, Yongin-si, Gyeonggi-do 16944 (KR); CHO, Hee Yeon, Seongnam-si, Gyeonggi-do 13486 (KR); YUN, Seok Kyun, Seongnam-si, Gyeonggi-do 13600 (KR); KANG, Seunghyun, Seoul 06285 (KR)
(74) Representative: Plougmann Vingtoft a/s
(86) International application number: PCT/KR2024/009339
(87) International publication number: WO 2025/009863

(57) **Abstract**

The present invention relates to a cosmetic composition for improving skin comprising honokiol and polyol, and a pharmaceutical composition for preventing or treating skin damage, wherein the honokiol improves skin wrinkles, improves elasticity, improves moisturizing, improves barrier, suppresses inflammation, and increases antioxidant activity when dissolved in polyol, and thus can be effectively used for manufacturing a cosmetic composition for improving skin or a pharmaceutical composition for preventing or treating skin damage.

## Description

### Technical field

This patent application claims priority to Korean Patent Application No.10-2023-0085480 submitted to the Korean Intellectual Property Office on July 3, 2023, and the disclosure of the patent application is inserted into this specification by reference.

The present invention relates to a cosmetic composition for improving skin comprising honokiol and polyol, and to a pharmaceutical composition for preventing or treating skin damage.

### Background of the invention

Aging is a functional, structural, and biochemical process that continuously occurs from birth to death of a human being, and occurs throughout the cells and body tissues that make up the human body, and it shows a decrease in metabolic rate, an increase in disease, and a decrease in adaptability, ultimately leading to death of cells and the entire body.

Among them, skin aging is largely divided into two categories, one is 'internal aging', which is an aging phenomenon due to an increase in age, and the other is 'external aging', which is an aging phenomenon due to external environments such as ultraviolet rays or pollution.

Several theories have been proposed so far regarding skin aging, but among them, the theory that approaches skin aging most scientifically is the theory of skin aging by oxidation.

The human skin is composed of the epidermis and dermis including the stratum corneum, and connective tissue, and the stratum corneum is composed of a dead cell layer formed through the differentiation process of keratinocyte, which is the base cell of the epidermis, and plays a role in protecting the human body from the influence of the external environment. In addition, the dermis layer existing inside the skin is composed of collagen and elastin, which gives elasticity to the skin and protects the skin from sagging. The antioxidant theory is a theory that collagen and elastin are damaged by free radicals generated by factors such as external ultraviolet rays, thereby reducing the elasticity of the skin and generating wrinkles.

Until now, vitamin E, vitamin C, carotenoid, glutathione, and plant extracts, which are natural antioxidants, have been used to prevent aging of the skin, especially wrinkles, but these substances have a low effect or cause skin irritation, redness, and inflammation, and have serious side effects on the skin.

Recently, as the trend of retinol products has increased, consumer needs have increased through the combination of strong wrinkle improvement effects and hypoallergenic effects such as retinol, but due to the limitation of improving side effects such as phototoxicity, the need for natural materials as retinol substitutes with low stimulation and excellent efficacy has emerged.

### Detailed Description of the Invention

### [Technical Challenge]

The present inventors have confirmed that skin elasticity enhancement, moisturization improvement, barrier improvement, inflammation alleviation, and antioxidant activity induced by honokiol and polyol are excellent.

Accordingly, an object of the present invention is to provide a cosmetic composition for improving skin comprising honokiol and polyol.

Another object of the present invention is to provide a pharmaceutical composition for preventing or treating skin damage, including honokiol and polyol. Another object of the present invention is to provide a use of honokiol and polyol for improving skin and a use of preventing or treating skin photoaging caused by UV exposure.

### [Technical Solution]

The present invention relates to a cosmetic composition for improving skin comprising honokiol, which is a component derived from magnolia bark, and a polyol, and a pharmaceutical composition for preventing or treating skin damage. According to the present invention, honokiol shows enhanced skin elasticity, moisturizing, barrier improvement, anti-inflammatory and antioxidant activity when dissolved in a polyol.

As a result of checking the activities of honokiol and polyol on skin cells, the present inventors confirmed that they exhibit more effective elasticity, moisturization, barrier improvement, inflammation suppression, and antioxidant effects compared to retinol, which is a known component.

Hereinafter, the present invention will be described in more detail.

An aspect of the present invention is a cosmetic composition for improving skin including honokiol and polyol.

In the present invention, the honokiol and the polyol may be included at a weight ratio of 1:10 to 1:200, and preferably at a weight ratio selected from 1:10 to 1:150, 1:10 to 1:100, 1:10 to 1:50, 1:10 to 1:20, 1:12 to 1:200, 1:12 to 1:150, 1:12 to 1:100, 1:12 to 1:50, 1:12 to 1:20, 1:15 to 1:200, 1:15 to 1:150, 1:15 to 1:100, 1:15 to 1:50, 1:15 to 1:20, 1:18 to 1:200, 1:18 to 1:150, 1:18 to 1:100, or 1:18 to 1:50, for example, at a weight ratio of 1:18 to 1:20, but the invention is not limited thereto.

In the present invention, the honokiol may be included in an amount of 0.0001 to 50 wt %, preferably 0.0001 to 20 wt %, 0.0001 to 10 wt %, 0.0001 to 5 wt %, 0.0001 to 2 wt %, 0.0001 to 1 wt %, 0.0001 to 0.5 wt %, 0.0001 to 0.2 wt %, 0.0001 to 0.1 wt %, 0.001 to 50 wt %, 0.001 to 20 wt %, 0.001 to 5 wt %, based on the total weight of the total cosmetic composition 0.001 to 5 wt %, 0.001 to 2 wt %, 0.001 to 1 wt %, 0.001 to 0.5 wt %, 0.001 to 0.2 wt %, 0.001 to 0.1 wt %, 0.01 to 50 wt %, 0.01 to 20 wt %, 0.01 to 10 wt %, 0.01 to 5 wt %, 0.01 to 2 wt %, 0.01 to 1 wt %, 0.01 to 0.5 wt %, 0.01 to 0.1 wt %, 0.01 to 0.1 wt %, 0.1 to 2 wt %, 0.01 to 0.1 wt %, 0.1 to 10 wt %, 0.1 to 5 wt %, 0.1 to 2 wt %, 0.1 to 1 wt %, 0.1 to 0.5 wt %, 0.1 to 0.2 wt %, 1 to 50 wt %, 1 to 20 wt %, 1 to 10 wt %, 1 to 5 wt %, 1 to 2 wt %, 2 to 50 wt %, 2 to 20 wt %, 2 to 10 wt %, 2 to 5 wt %, 5 to 50 wt %, 5 to 20 wt %, 5 to 10 wt %, 10 to 50 wt %, or 10 to 20 wt %, but it is not limited thereto.

In the present invention, the polyol may be one or more selected from the group consisting of Butylene Glycol (BG) and Propylene Glycol (PG), preferably butylene glycol, but is not limited thereto.

In the present invention, the skin improvement may be an improvement against skin photoaging caused by exposure to ultraviolet rays.

The ultraviolet rays may be, for example, ultraviolet-A (UVA) or ultraviolet-B (UVB). However, the disclosure is not limited to the above examples, and includes all light in a wavelength range that may cause skin photoaging.

In the present invention, the skin improvement may be at least one selected from the group consisting of skin wrinkle improvement, elasticity improvement, moisturization improvement, barrier improvement, inflammation suppression, and antioxidant.

The cosmetic composition of the present invention may be prepared in any formulation conventionally prepared in the art, and may be applied to various functional cosmetic compositions such as an aging functional cosmetic, a whitening functional cosmetic, and the like in addition to general cosmetic compositions such as emulsions, lotions, essences, and creams.

In addition, the cosmetic composition may be prepared in various formulations without particular limitation, for example, in the form of a cream, toner, emulsion, or essence. More specifically, it may be formulated as a softening toner, nourishing toner, essence, nourishing emulsion, nourishing cream, eye cream, massage cream, cleansing cream, cleansing foam, cleansing water, powder, mask, hair care product, body emulsion, body cream, body oil, body essence, or body cleanser.

In a preferred embodiment, the cosmetic composition of the present invention may be a formulation of a cream, emulsion or essence. The carrier or additive used in this case may be selectively used according to the formulation of the cosmetic.

Therefore, the cosmetic composition used in the present invention may further include, in addition to the above ingredients, ingredients that may be generally used in cosmetics such as creams, emulsions, essences, and the like, for example, maintenance ingredients, moisturizers, emollients, surfactants, organic and inorganic pigments, organic powders, ultraviolet absorbers, preservatives, disinfectants, antioxidants, plant extracts, pH adjusting agents, alcohols, pigments, fragrances, blood circulation promoters, cold sensitizers, antiperspirants, preservatives, and solvents, and may further add fragrances according to preference.

In addition, when the formulation of the present invention is a paste, cream, or gel, animal fibers, plant fibers, waxes, paraffin, starch, tracant, cellulose derivatives, polyethylene glycol, silicone, bentonite, silica, talc, or zinc oxide may be used as carrier components. When the formulation of the present invention is a powder or a spray, lactose, talc, silica, aluminum hydroxide, calcium silicate or polyamide powder may be used as a carrier component, and in particular, when the formulation is a spray, a propellant such as chlorofluorohydrocarbon, propane/butane or dimethyl ether may be additionally included.

When the formulation of the present invention is a solution or an emulsion, a solvent, a solvating agent, or an emulsifying agent is used as a carrier component, for example, water, ethanol, isopropanol, ethyl carbonate, ethyl acetate, benzyl alcohol, benzylbenzoate, propylene glycol, 1,3-butyl glycol oil, glycerol aliphatic ester, polyethylene glycol, or fatty acid ester of sorbitan. When the formulation of the present invention is a suspension, liquid diluents such as water, ethanol or propylene glycol, suspensions such as ethoxylated isostearyl alcohol, polyoxyethylene sorbitol ester and polyoxyethylene sorbitan ester, microcrystalline cellulose, aluminum metahydroxide, bentonite, agar or tragacanth may be used as carrier components.

Another aspect of the present invention is a pharmaceutical composition for preventing or treating skin damage, including honokiol and polyol.

In the present invention, the honokiol and the polyol may be included in a weight ratio of 1:10 to 1:200, preferably 1:10 to 1:150, 1:10 to 1:100, 1:10 to 1:50, 1:10 to 1:20, 1:12 to 1:200, 1:12 to 1:150, 1:12 to 1:100, 1:12 to 1:50, 1:12 to 1:20, 1:15 to 1:200, 1:15 to 1:150, and 1:10 to 10 It may be included in a weight ratio of 1:50, 1:15 to 1:20, 1:18 to 1:200, 1:18 to 1:150, 1:18 to 1:100, or 1:18 to 1:50, for example, may be included in a weight ratio of 1:18 to 1:20, but is not limited thereto.

In the present invention, the honokiol may be included in an amount of 0.0001 to 50 wt %, preferably 0.0001 to 20 wt %, 0.0001 to 10 wt %, 0.0001 to 5 wt %, 0.0001 to 2 wt %, 0.0001 to 1 wt %, 0.0001 to 0.5 wt %, 0.0001 to 0.2 wt %, 0.0001 to 0.1 wt %, 0.001 to 50 wt %, 0.001 to 20 wt %, 0.001 to 5 wt %, based on the total weight of the total cosmetic composition 0.001 to 5 wt %, 0.001 to 2 wt %, 0.001 to 1 wt %, 0.001 to 0.5 wt %, 0.001 to 0.2 wt %, 0.001 to 0.1 wt %, 0.01 to 50 wt %, 0.01 to 20 wt %, 0.01 to 10 wt %, 0.01 to 5 wt %, 0.01 to 2 wt %, 0.01 to 1 wt %, 0.01 to 0.5 wt %, 0.01 to 0.1 wt %, 0.01 to 0.1 wt %, 0.1 to 2 wt %, 0.01 to 0.1 wt %, 0.1 to 10 wt %, 0.1 to 5 wt %, 0.1 to 2 wt %, 0.1 to 1 wt %, 0.1 to 0.5 wt %, 0.1 to 0.2 wt %, 1 to 50 wt %, 1 to 20 wt %, 1 to 10 wt %, 1 to 5 wt %, 1 to 2 wt %, 2 to 50 wt %, 2 to 20 wt %, 2 to 10 wt %, 2 to 5 wt %, 5 to 50 wt %, 5 to 20 wt %, 5 to 10 wt %, 10 to 50 wt %, or 10 to 20 wt %, but it is not limited thereto.

In the present invention, the polyol may be one or more selected from the group consisting of butylene glycol and propylene glycol, preferably butylene glycol, but is not limited thereto.

In the present invention, the skin damage may be skin photoaging caused by exposure to ultraviolet rays.

The ultraviolet rays may be, for example, UVA or UVB. However, the disclosure is not limited to the above examples, and includes all light in a wavelength range that may cause skin photoaging.

In the present invention, the skin damage caused by photoaging may be at least one selected from the group consisting of skin wrinkles, skin elasticity reduction, skin dryness, loss of skin barrier function, and skin inflammation.

The pharmaceutical composition of the present invention may be used as a pharmaceutical composition comprising a pharmaceutically effective amount of honokiol and/or a pharmaceutically acceptable carrier.

In this specification, the term "pharmaceutically effective amount" means an amount sufficient to achieve the above-described efficacy or activity of honokiol.

The pharmaceutically acceptable carrier included in the pharmaceutical composition of the present invention is commonly used in preparation, and includes lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, and the like, but is not limited thereto. The pharmaceutical composition of the present invention may further include a lubricant, a wetting agent, a sweetener, a flavoring agent, an emulsifier, a suspending agent, a preservative, and the like in addition to the above components.

The pharmaceutical composition according to the present invention may be administered to mammals including humans in various routes. The administration method may be any method commonly used, for example, by oral, skin, vein, muscle, subcutaneous route, and preferably by oral administration.

Suitable dosages of the pharmaceutical compositions of the present invention vary depending on factors such as formulation method, administration mode, age, weight, sex, pathological condition, food, administration time, route of administration, excretion rate, and response sensitivity of the patient, and ordinarily skilled physicians can readily determine and prescribe an effective dosage for the desired treatment or prevention.

The pharmaceutical composition of the present invention may be prepared in a unit dose form by being formulated using a pharmaceutically acceptable carrier and/or excipient, or may be prepared by being inserted into a multi-dose container, according to a method that can be easily carried out by a person having ordinary skill in the art to which the present invention belongs. In this case, the formulation may be in the form of a solution, a suspension, or an emulsion in an oil or aqueous medium, or in the form of an X agent, a powder, a granule, a tablet, a capsule or a gel (e.g., a hydrogel), and may further include a dispersing agent or a stabilizing agent.

### [Effects of Invention]

The present invention relates to a cosmetic composition for improving skin comprising honokiol and polyol, and a pharmaceutical composition for preventing or treating skin damage, wherein the honokiol improves skin wrinkles, improves elasticity, improves moisturizing, improves barrier, suppresses inflammation, and increases antioxidant activity when dissolved in polyol, and thus can be effectively used for manufacturing a cosmetic composition for improving skin or a pharmaceutical composition for preventing or treating skin damage.

### Brief description of the drawings

FIG. 1A is a graph showing cytotoxicity of honokiol to skin stratum corneum cells according to an embodiment of the present invention.
FIG. 1B is a graph showing cytotoxicity of honokiol against skin dermal cells according to an embodiment of the present invention.
FIG. 2A is a graph showing the expression level of the gene COL1A related to promoting collagen production in honokiol according to an embodiment of the present invention.
FIG. 2B is a graph showing the expression level of the gene MMP-1 related to inhibition of collagen degradation in honokiol according to an embodiment of the present invention.
FIG. 3A is a graph showing the expression level of the gene ELN1 related to promoting elastin production in honokiol according to an embodiment of the present invention.
FIG. 3B is a graph showing the expression level of the gene FBN related to promoting fibronectin production in honokiol according to an embodiment of the present invention.
FIG. 4A is a graph showing the expression amount of the gene AQP3 related to the movement of moisture present in the dermis in honokiol according to an embodiment of the present invention.
FIG. 4B is a graph showing the expression level of the gene HAS3 related to hyaluronic acid synthase in honokiol according to an embodiment of the present invention.
FIG. 5A is a graph showing the expression level of the gene FLG related to moisturizing component present in keratinocytes constituting a skin barrier in honokiol according to an embodiment of the present invention.
FIG. 5B is a graph showing the expression level of the gene CLDN1 related to skin barrier binding in honokiol according to an embodiment of the present invention.
FIG. 6 is a graph showing the expression level of the gene TSLP related to inflammation expressed in skin keratinocytes in honokiol according to an embodiment of the present invention.
FIG. 7 is a graph showing the antioxidant activity of honokiol according to an embodiment of the present invention.
FIG. 8A is a graph showing the expression level of the gene COL1A related to promoting collagen production according to the solvent selection of honokiol according to an embodiment of the present invention.
FIG. 8B is a graph showing the expression level of the gene MMP-1 related to inhibition of collagen degrading enzyme according to the solvent selection of honokiol according to an embodiment of the present invention.
FIG. 9A is a graph showing the expression level of the gene ELN1 related to elastin production promotion according to the solvent selection of honokiol according to an embodiment of the present invention.
FIG. 9B is a graph showing the expression level of FLG, which is a factor related to the promotion of epidermal cell formation according to the selection of a solvent of honokiol according to an embodiment of the present invention.

### Best mode for carrying out the invention

The present invention relates to a cosmetic composition for improving skin comprising honokiol and polyol.

### Embodiments of the invention

Hereinafter, the present invention will be described in more detail by the following examples. However, these examples are only for illustrating the present invention, and the scope of the present invention is not limited by these examples.

Throughout this specification, the %" used to indicate the concentration of a particular substance is (weight/weight) % of solid/solid, (weight/volume) % of solid/liquid, and (volume/volume) % of liquid/liquid, unless otherwise stated.

### Examples

### Example 1: Evaluation of Cytotoxicity

Toxicity assessments of honokiol on skin epidermis and dermal cells were performed.

Specifically, HaCaT cells, which are skin keratinocytes, and Hs68 cells, which are skin dermis cells, were cultured in a 96 well plate at a concentration of 2 X 104/well and cultured in a medium including FBS (Federal Bovine Serum) and antibiotics (Antibiotic antibiotic) at 37°C and 5% CO₂ for 24 hours, and then further cultured by treating the medium including only antibiotics with 0.1, 0.5, 1, and 5 ppm for each concentration on the next day. After 24 hours, the toxic concentration of the honokiol material on skin cells was determined by replacing it with medium containing MTT tetrazolum (Sigma-aldrich, St. Lous, USA) and by reacting at 37°C for 4 hours to evaluate the cellular metabolic activity.

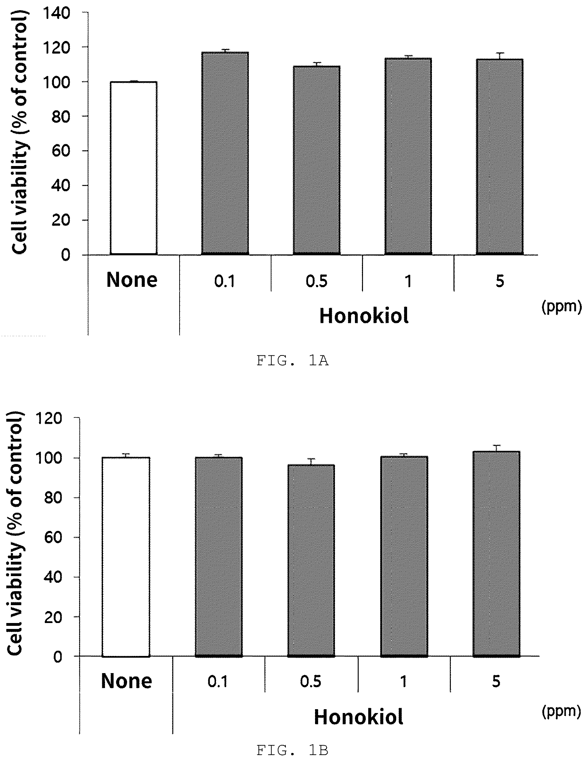

As can be seen in FIGS. 1A and 1B, honokiol did not cause cytotoxicity at concentrations of 5 ppm or less for both skin stratum corneum cells and skin dermis cells.

### Example 2: Confirmation of Effect of Honokiol

### 2-1. Identification of Collagen Production

It was determined whether honokiol increases the amount of skin collagen. Specifically, Hs68 cells, which are skin dermal layer cells, were irradiated with 25 mJ/cm² intensity of UVB (ultraviolet B), and then treated with RA (retinoic acid), EGCG (epigallocatechin gallate), retinol, and honokiol at a concentration of 1 ppm for 24 hours, respectively.

The mRNA expression of the collagen production promotion factor COL1A1 and the collagen degradation enzyme factor MMP-1 gene was measured through qRT-PCR analysis and converted based on the actin expression, and the relative values for the expression of the UVB non-irradiated group were calculated and compared.

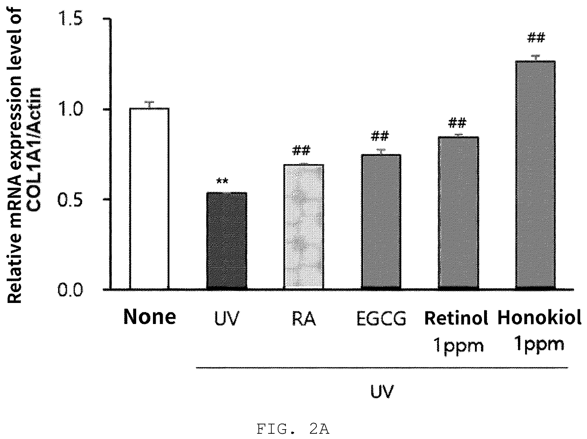

As shown in FIG. 2A, the honokiol-treated group showed a higher collagen production than the UVB-only irradiated group, and showed the highest collagen production among all UVB irradiated groups.

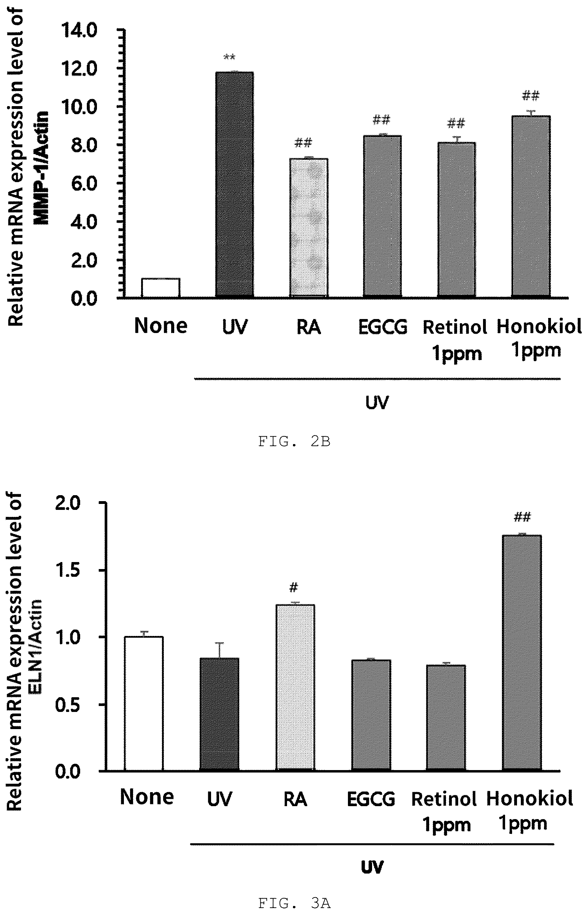

As can be seen in FIG. 2B, the honokiol-treated group showed a lower amount of collagen degrading enzyme production than the UVB-only irradiation group.

As a result, it was found that honokiol has the effect of increasing the skin collagen content by promoting collagen production and inhibiting the degradation of collagen.

### 2-2. Confirmation of the Effect of Increasing Elasticity

It was confirmed whether honokiol increases skin elasticity.

Specifically, Hs68 cells, which are skin dermal layer cells, were irradiated with UVB (ultraviolet-B) having a 25 mJ/cm² intensity, and then treated with RA, EGCG, retinol, and honokiol, 1 ppm each, for 24 hours.

The mRNA expression levels of ELN1, a factor associated with elastin production promotion, and FBN, a factor associated with fibronectin production promotion, were then measured using qRT-PCR analysis, and the levels of actin expression from the UVB non-irradiated group were converted and compared.

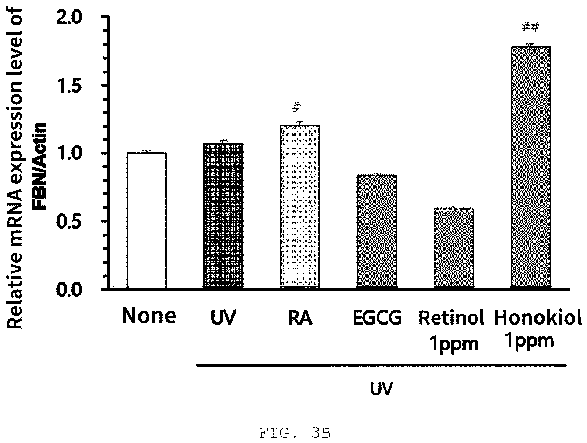

As can be seen in FIGS. 3A and 3B, the honokiol-treated group not only showed the highest expression level among the UV-irradiated groups but also showed a higher expression level than the UV-unirradiated group.

Consequently, honokiol was found to increase skin elasticity by promoting the production of elastin and fibronectin, the skin components responsible for elasticity.

### 2-3. Confirmation of Moisturizing Improvement Effect

It was checked whether honokiol improves skin moisturization.

Specifically, HaCaT cells, which are skin stratum corneum cells, were treated with retinol and honokiol at 0.1 and 1 ppm, respectively, for 24 hours.

The mRNA expression levels of the AQP3 gene, which is a factor related to the migration of moisture present in the dermis, and the HAS3 gene, which is a factor related to hyaluronic acid synthase, were then measured through qRT-PCR analysis, and the β-actin expression levels were converted and compared.

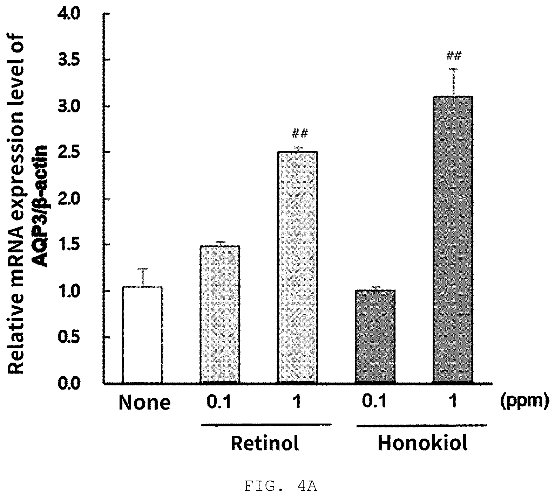

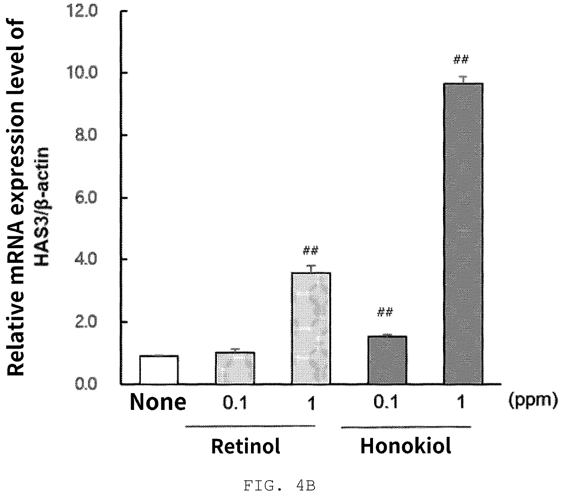

As can be seen in FIGS. 4A and 4B, the honokiol-treated group not only showed a higher overall expression than the retinol-treated group, but also significantly increased the expression of related genes, especially during 1 ppm treatment.

Consequently, honokiol was found to improve skin moisturization by activating water channels and promoting hyaluronic acid synthesis.

### 2-4. Identification of Barrier Improvement Effects

It was determined whether honokiol improves the skin barrier.

Specifically, HaCaT cells, which are skin stratum corneum cells, were treated with retinol and honokiol at 0.1 and 1 ppm, respectively, for 24 hours.

Subsequently, through qRT-PCR analysis, the mRNA expression of filaggrin (FLG), a moisturizing component-related factor present in keratinocytes constituting the skin barrier, and the CLDN1 gene, a factor related to the binding of the skin barrier, were measured and compared based on the β-actin expression level.

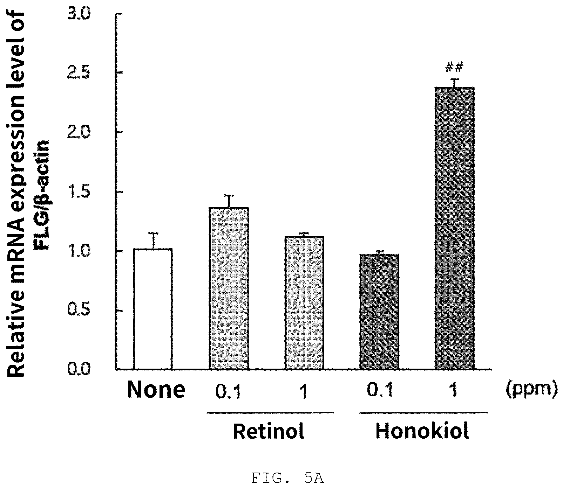

As can be seen in FIG. 5A, the honokiol-treated group not only showed higher expression than the retinol-treated group overall, but also significantly increased the expression of the FLG gene especially during 1 ppm treatment.

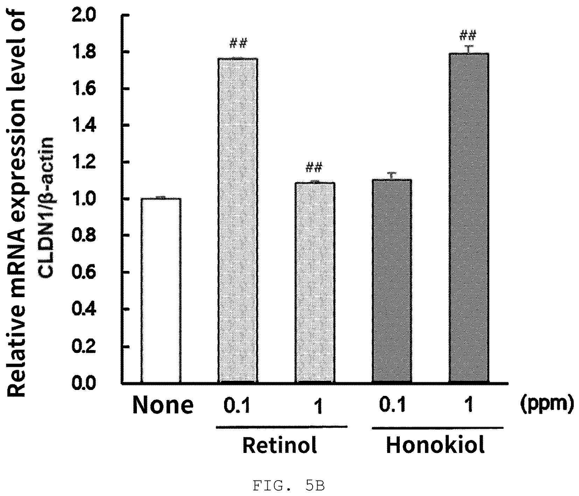

As can be seen in FIG. 5B, the honokiol-treated group significantly increased the expression level of the CLDN1 gene compared to that of the control group, especially when treated with 1 ppm.

As a result, it was found that honokiol improves skin moisturization by promoting differentiation of the skin barrier and strengthening the binding between the skin barriers.

### 2-5. Confirmation of the Effect of Relieving Epidermal Inflammation

It was determined whether honokiol relieves inflammation of the skin epidermis.

Specifically, HaCaT cells, which are skin stratum corneum cells, were treated (+) with 10 µg/ml of Poly I:C and 10 µg/ml of IL-4 protein, which are inflammatory response inducing substances, and prepared as an experimental group in which 1 µM (Dexa) of dexamethasone (DEXA), retinol, and honokiol, which are control inflammatory response inducing substances, were treated for 4 hours at 0.1 and 1 ppm, respectively. The untreated group (-) was prepared as a negative control.

Afterwards, the expression level of the TSLP gene, an inflammation-related factor expressed in skin keratinocytes, was measured through qRT-PCR analysis, converted based on the expression level of ectin, and compared.

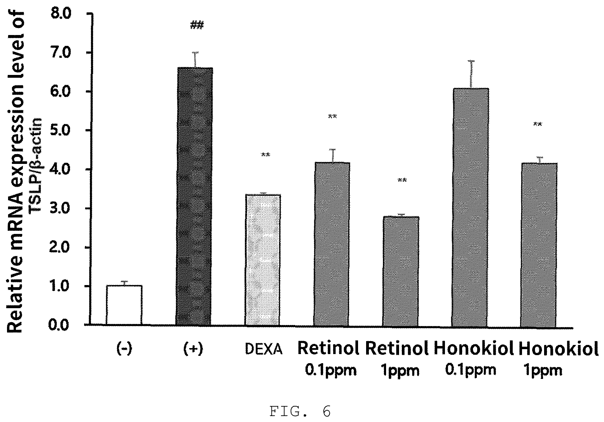

As can be seen in FIG. 6, in cells in which an inflammatory response is induced, the honokiol-treated group was found to exert an effect of alleviating inflammation of the epidermis by significantly reducing the expression amount of the TSLP gene when treated with 1 ppm.

### 2-6. Confirmation of Antioxidant Effects

It was confirmed by the DPPH test whether honokiol exerts antioxidant activity.

Specifically, the DPPH solution was treated with Ascorbic Acid (AA), retinol, and honokiol at 10 and 100 ppm, respectively, at room temperature for 30 minutes. The absorbance was measured at 517 nm to show antioxidant activity as the ratio of the decrease in absorbance to the control. At this time, ascorbic acid, an antioxidant, was used as a control group for determining the absorbance reduction ratio.

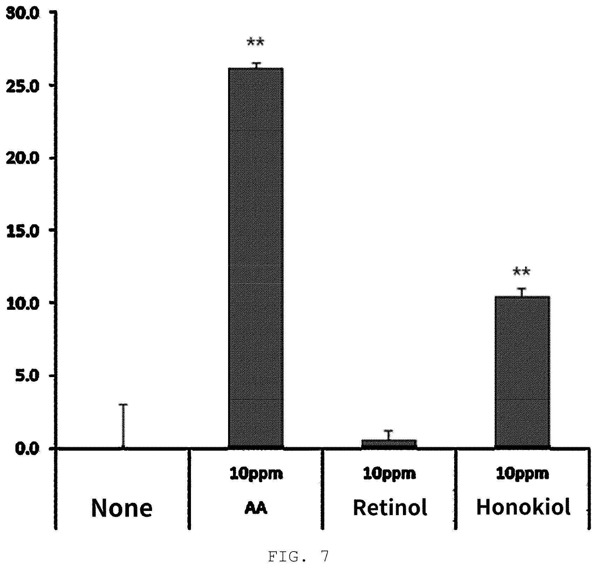

As can be seen in FIG. 7, it was confirmed that the honokiol-treated group exhibited remarkably high antioxidant activity compared to retinol.

### Example 3: Stabilization Method of High Concentration Honokiol

### 3-1. Selection of Solvents

Honokiol is a poorly soluble component and has low solubility in water, so it has the property of being dissolved in an organic solvent. To stabilize the cosmetic composition including honokiol at a high concentration of 5 wt %, three types of solvents corresponding to water, Butylene Glycol (BG), and medium-chain triglyceride (MCT) oil were applied to confirm the result.

**[Table 1]**

| OD | Color | Odor | Precipitation |
|---|---|---|---|
| 5% Honokiol (BG) | Light brown | X | X |
| 5% Honokiol (MCT) | Light yellow | X | X |

As shown in Table 1, as a result of dissolving 5 wt % of honokiol, the solubility in water was low, but it was found to be well soluble in BG and MCT.

### 3-2. Stability and Potency Testing with Selected Solvent Applications

Honokiol was dissolved in BG and MCT at a concentration of 5 wt %, and high-performance liquid chromatography (HPLC) was performed to observe whether precipitates were formed at 4°C, 25°C, 45°C, 50°C, and sunlight, and to analyze potency.

**[Table 2]**

| | 5% Honokiol (BG) | | 5% Honokiol (MCT) | |
|---|---|---|---|---|
| 4°C | | 5.17 | | 5.13 |
| 25°C | 5.19 | 5.12 | 5.14 | 5.07 |
| 45°C | | 5.10 | | 5.03 |
| Cycle | | 5.11 | | 5.03 |

As can be seen in Table 2, since honokiol maintained its potency well even in harsh environments of low temperature, high temperature, and cycle, it could be judged that the stability of the material itself was excellent.

Subsequently, after preparing the same composition, it was confirmed whether or not precipitates were produced at 4°C, 25°C, 45°C, 50°C, and sunlight for 3 months.

**[Table 3]**

| 1M | Color | | Odor | Precipitation |
|---|---|---|---|---|
| 5% Honokiol (BG) | 25°C | Light brown | X | X |
| | 4°C | Light brown | X | X |
| | 45°C | Brown | X | X |
| | 50°C | Brown | X | X |
| | Light | Light brown | X | X |
| 5% Honokiol (MCT) | 25°C | Light yellow | X | Δ |
| | 4°C | Light yellow | X | X |
| | 45°C | Yellow | X | O |
| | 50°C | Yellow | X | O |
| | Light | Light yellow | X | Δ |

As can be seen in Table 3, when BG and MCT were used as solvents, overall stability was shown under all conditions, but in the case of MCT, precipitation occurred under high temperature conditions as different patterns compared to BG after 1 month (1M), and signs of precipitation were found under 25°C and sunlight conditions. This was determined to be due to the high stability of the honokiol component itself but the low dissolution stability at MCT. There was no significant difference in characteristics from the results after 3 months and after 1 month.

In conclusion, it was confirmed that the poorly soluble honokiol exhibited higher solubility stability in the hydrophilic polyol than in the lipophilic oil.

### Example 4: Confirmation of Effect by Combination of Honokiol and Solvent

### 4-1. Identification of Collagen Production

The collagen production amount was compared in the same manner as in Example 2-1, and the treatment sample was changed to a honokiol solution dissolved in RA (retinoic acid), retinol, DMSO (dimethyl sulfoxide), a honokiol solution dissolved in BG, and a honokiol solution dissolved in PG (propylene glycol).

In Example 2-1, a 5% (w/w) solution was prepared as a sample to confirm stability in a high concentration dissolved state, whereas a honokiol solution for treating cells was prepared at a concentration of 1 mg/ml, and the honokiol alone component treated in cells was performed to be 1 ppm.

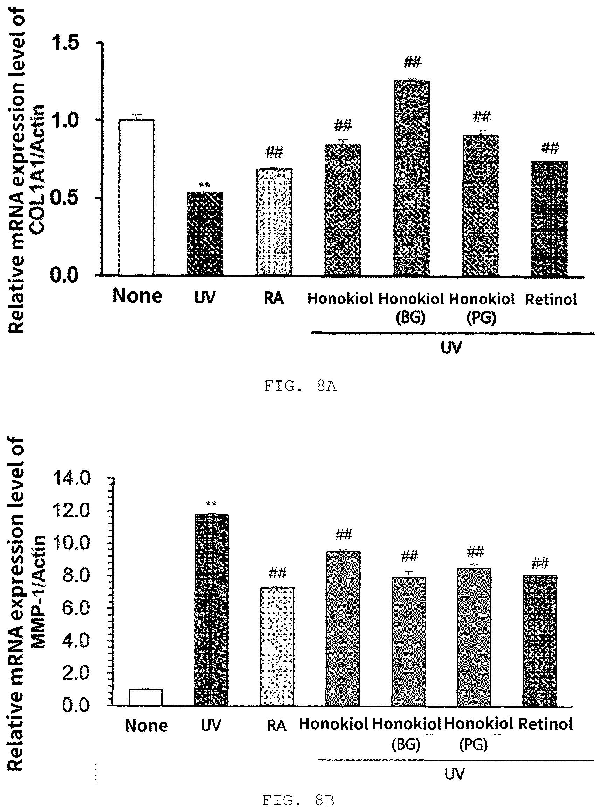

As can be seen in FIG. 8A, the honokiol solution treatment group dissolved in BG showed higher collagen production than the UVB-only irradiation group, and showed the highest collagen production among all UVB irradiation groups.

As can be seen in FIG. 8B, all groups treated with the honokiol solution showed lower collagen degrading enzyme production than the group irradiated with UVB alone.

### 4-2. Confirmation of the Effect of Increasing Elasticity

The degree of increase in skin elasticity was compared in the same manner as in Example 2-2, and the mRNA expression levels of the ELN1 gene, which is a factor related to elastin production promotion, and the FLG gene, which is a factor related to epidermal cell production promotion, were measured and converted based on the actin expression level from the UVB non-irradiated group and compared.

The treatment sample was prepared in the same manner as in Example 4-1 with RA, retinol, a honokiol solution dissolved in dimethyl sulfoxide (DMSO), a honokiol solution dissolved in BG, and a honokiol solution dissolved in Propylene Glycol (PG), and was performed so that the concentration of honokiol treated in the cells was 1 ppm.

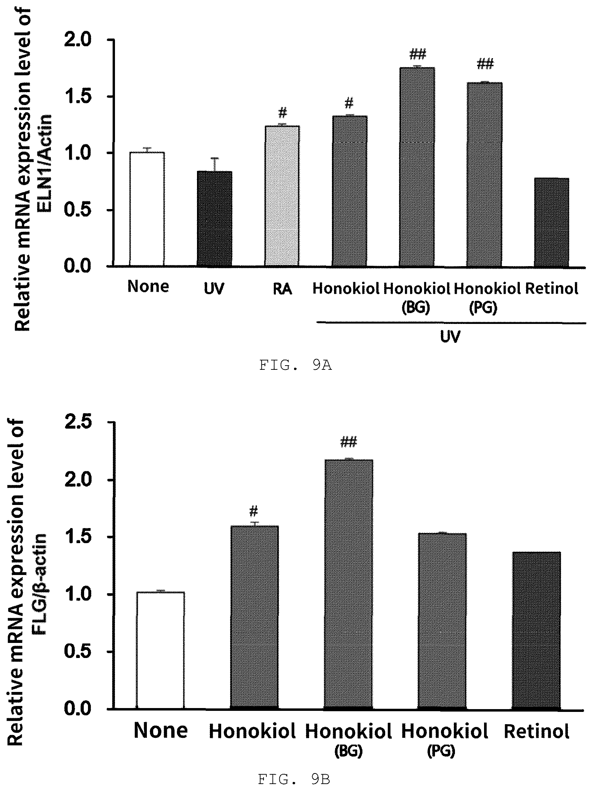

As can be seen in FIGS. 9A and 9B, the BG-dissolved honokiol solution treatment group not only showed the highest expression level among the UV irradiation group but also showed higher expression level than the UV non-irradiation group, and showed the highest expression level among all UVB irradiation groups.

Consequently, honokiol was found to increase skin elasticity by promoting the production of elastin and fibronectin, the skin components responsible for elasticity.

### Industrial Applicability

The present invention relates to a cosmetic composition for improving skin comprising honokiol and polyol, and to a pharmaceutical composition for preventing or treating skin damage.

## Claims

1. A cosmetic composition for improving skin comprising honokiol and polyol.

2. The cosmetic composition of claim 1, wherein the honokiol is included in an amount of 0.0001 to 50 wt % based on the total weight of the cosmetic composition.

3. The cosmetic composition of claim 1, wherein the polyol is at least one selected from the group consisting of Butylene Glycol (BG) and Propylene Glycol (PG).

4. The cosmetic composition of claim 1, wherein the skin improvement is an improvement on skin photoaging caused by exposure to ultraviolet rays.

5. The cosmetic composition of claim 1, wherein the skin improvement is at least one selected from the group consisting of skin wrinkle improvement, elasticity improvement, moisturization improvement, barrier improvement, inflammation inhibition, and antioxidant.

6. A pharmaceutical composition for preventing or treating skin damage, comprising honokiol and polyol.

7. The cosmetic composition of claim 6, wherein the honokiol is included in an amount of 0.0001 to 50 wt % based on the total weight of the cosmetic composition.

8. The cosmetic composition of claim 6, wherein the polyol is at least one selected from the group consisting of Butylene Glycol (BG) and Propylene Glycol (PG).

9. The pharmaceutical composition of claim 6, wherein the skin damage is skin photoaging caused by exposure to ultraviolet light.

10. The pharmaceutical composition of claim 6, wherein the skin damage is at least one selected from the group consisting of skin wrinkles, reduced skin elasticity, dry skin, loss of skin barrier function, and skin inflammation.
